(19) Europäisches Patentamt
European Patent Office
Office européen des brevets

(11) Veröffentlichungsnummer: **0 004 635**
**B1**

(12) **EUROPÄISCHE PATENTSCHRIFT**

(45) Veröffentlichungstag der Patentschrift: **12.08.81**

(51) Int. Cl.³: **C 07 C 99/00**, C 07 C 101/54

(21) Anmeldenummer: **79100927.7**

(22) Anmeldetag: **28.03.79**

# (54) Verfahren zur kontinuierlichen Herstellung von Anthranilsäure.

(30) Priorität: **11.04.78 DE 2815522**

(43) Veröffentlichungstag der Anmeldung: **17.10.79 Patentblatt 79/21**

(45) Bekanntmachung des Hinweises auf die Patenterteilung: **12.08.81 Patentblatt 81/32**

(84) Benannte Vertragsstaaten: **BE CH DE FR GB**

(56) Entgegenhaltungen: **DE-A-2 000 698**

(73) Patentinhaber: **BASF Aktiengesellschaft, Carl-Bosch-Strasse 38, D-6700 Ludwigshafen (DE)**

(72) Erfinder: **Kilpper, Gerhard, Dr., Waldstrasse 3, D-6719 Battenberg (DE)**
Erfinder: **Grimmer, Johannes, Duererstrasse 12, D-6700 Ludwigshafen (DE)**

**EP 0 004 635 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur kontinuierlichen Herstellung von Anthranilsäure

Die Erfindung betrifft ein Verfahren zur kontinuierlichen Herstellung von Anthranilsäure durch Umsetzung von phthalamidsaurem und/oder phthalimidsaurem Alkali mit Alkalihypohalogeniten in einem Reaktionsrohr, wobei man während oder nach der Reaktion die erhaltene Anthranilsäure in zwei Schritten mit unterschiedlichen Reduktionsmitteln behandelt und im zweiten Behandlungsschritt als Reduktionsmittel Alkalidithionit der Alkaliformaldehydsulfoxylat verwendet.

Es ist aus der deutschen Patentschrift 1 224 748 bekannt, daß man phthalamidsaures Alkali durch Oxidation mit Alkalihypochlorit kontinuierlich zur Anthranilsäure umsetzt. Die Ausgangsstoffe werden in Form ihrer gekühlten, wäßrigen Lösungen miteinander in einer gekühlten Mischkammer vermischt und in der ersten Reaktionsstufe, der Bildung von Phenylisocyanat-2-carbonsäure, in dem ein Kühlsystem enthaltenden Teil einer Reaktionskolonne umgesetzt. In der zweiten Stufe, der Bildung von Anthranilsäure, soll die Reaktionstemperatur 70° C nicht übersteigen.

Aus den deutschen Auslegeschriften 1 950 281 und 2 000 698 ist ein Verfahren zur kontinuierlichen Herstellung von Anthranilsäure durch Umsetzung von phthalamidsaurem und/oder phthalimidsaurem Alkali mit Hypohalogeniten in wäßrigem Medium bekannt, wobei man

a) eine wäßrige Lösung von phthalamidsaurem und/oder phthalimidsaurem Alkali und eine wäßrige Lösung von Alkalihypochlorit in einer Mischvorrichtung mischt,
b) die erhaltene Mischung im ersten Teil eines engen Reaktionsrohres mit hoher Strömungsgeschwindigkeit bei 10 bis 50° C unter weitgehend adiabatischen Bedingungen umsetzt, anschließend
c) die aus dem ersten Teil des Reaktionsrohres mit hoher Strömungsgeschwindigkeit abströmende Umsetzungsmischung im zweiten Teil des genannten Rohres bei 60 bis 80° C zu Ende umsetzt und
d) aus der abströmenden alkalischen Umsetzungsmischung in üblicher Weise Anthranilsäure abtrennt, und gegebenenfalls während der Stufe b) und/oder Stufe c) ein Reduktionsmittel zusetzt.

Bei diesem Verfahren wird ein Teil des freien Alkalihydroxids schon beim Lösen des Ausgangsstoffes verwendet, ein weiterer Teil der Hypochloritlösung zugesetzt. Vorteilhaft gelangen wäßrige Lösungen von 10 bis 50 Gewichtsprozent Phthalimid und/oder Phthalamid zur Anwendung, die von 1 bis 1,1 Mol Alkalihydroxid je Mol Phthalimid/Phthalamid enthalten. Es wird angegeben, daß die wäßrigen Hypohalogenitlösungen vorteilhaft von 8 bis 15 Gewichtsprozent Hypohalogenit und von 0 bis 3, vorzugsweise von 0,02 bis 2,1 Mol Alkalihydroxid je Mol Phthalimid/Phthalamidsäure enthalten. Die deutsche Auslegeschrift 2 000 698 verwendet ausdrücklich nur ein Reduktionsmittel, bevorzugt Natriumsulfit und Natriumbisulfit (siehe auch Beispiel); bevorzugt wird es nur an einer Stelle dem Umsetzungsgemisch zugesetzt, vorteilhaft direkt nach Beendigung der Umsetzung in Stufe b).

Die deutsche Offenlegungsschrift 2 328 757 beschreibt ein Verfahren zur Herstellung von Aminen, z. B. Anthranilsäure, durch Umsetzung von Carbonsäureamiden mit Hypochloriten in Gegenwart von Brom, Jod und/oder Halogenamiden und überschüssigem Alkalihydroxid. Es wird angegeben, daß vorteilhaft wäßrige Suspensionen von 1 bis 50 Gewichtsprozent Ausgangscarbonsäureamid zur Anwendung gelangen. Die wäßrigen Hypochloritlösungen enthalten im allgemeinen von 5 bis 15, vorzugsweise von 12 bis 14 Gewichtsprozent Hypochlorit und können zusätzlich von 0,2 bis 2,5 Mol, vorzugsweise 1 bis 2,1 Mol Alkalihydroxid je Mol Hypochlorit enthalten. Im Ausgangsgemisch beider Ausgangsstoffe kommen im allgemeinen Mengen von insgesamt 0,2 bis 2,5 Mol, vorzugsweise 1 bis 2,1 Mol Alkalihydroxid (nicht eingerechnet das im Hypochlorit enthaltene Alkali), bezogen auf 1 Mol Ausgangscarbonsäureamid und Carbonamidgruppe am Molekül, in Frage. Enthält die wäßrige Hypochloritlösung kein freies Alkalihydroxid, so werden am Anfang oder im Laufe der Umsetzung zweckmäßig von 0,2 bis 2, vorzugsweise 1 bis 2 Mol Alkalihydroxid pro Mol Hypochlorit zugeführt.

Ein weiteres in der deutschen Offenlegungsschrift 2 357 749 beschriebenes Verfahren zeigt die entsprechende Herstellung von Aminen durch Umsetzung von Carbonsäureamiden mit Hypochloriten in Gegenwart von überschüssigem Alkalihydroxid und in Gegenwart von Polymerisationsinhibitoren. Vorteilhaft verwendet man wäßrige Suspensionen von 1 bis 50 Gewichtsprozent Ausgangscarbonsäureamid, wäßrigen Hypochloritlösungen und Mengen an Alkalihydroxid je Mol Hypochlorit wie bei dem Verfahren der deutschen Offenlegungsschrift 2 328 757. Enthält die wäßrige Hypochloritlösung kein freies Alkalihydroxid, so werden am Anfang oder im Laufe der Umsetzung ebenfalls zweckmäßig von 0,2 bis 2, vorzugsweise 1 bis 2 Mol Alkalihydroxid pro Mol Hypochlorit zugeführt.

Für manche Verwendungszwecke, z. B. Folgeprodukte auf dem Pharmasektor, ist es erforderlich, Anthranilsäure zu verwenden, die in verdünnter Salzsäure wasserhell und ohne Rückstände löslich ist und einen Gehalt von mindestens 99 Prozent aufweist. Bei der Herstellung von Anthranilsäure können intensiv rot bis gelb gefärbte Verbindungen entstehen, die bei der Fällung der Anthranilsäure aus der Lösung adsorbiert werden und beim Auflösen eine unerwünschte Färbung verursachen. Wenn auch das in der deutschen Auslegeschrift 2 000 698 beschriebene Verfahren eine vergleichsweise reinere Anthranilsäure herstellen läßt, so sind alle diese Verfahren mit Bezug auf die Herstellung besonders

reiner Anthranilsäure unbefriedigend. Zusätzliche Verfahren zur Reinigung wie Filtration unter Zusatz von Aktivkohle oder Umfällung sind technisch umständlich, aufwendig und außerdem mit Verlust an Endstoff verbunden.

Es wurde nun gefunden, daß man kontinuierlich Anthranilsäure durch Umsetzung von phthalamidsaurem und/oder phthalimidsaurem Alkali mit Hypohalogeniten in wäßrigem Medium in einem Reaktionsrohr und unter Behandlung des die gebildete Anthranilsäure enthaltenden Reaktionsgemisches mit Reduktionsmitteln vorteilhaft erhält, wenn man während der Reaktion oder nach Beendigung der Reaktion die Behandlung mit Reduktionsmitteln in 2 Schritten durchführt, wobei man in einem ersten Schritt das Reaktionsgemisch, das die erhaltene Anthranilsäure enthält, mit den Alkalidithionit und/oder Alkaliformaldehydsulfoxylat unterschiedlichen Reduktionsmitteln behandelt und in einem zweiten Schritt die behandelte Anthranilsäure mit Alkalidithionit und/oder Alkaliformaldehydsulfoxylat behandelt.

Die Umsetzung läßt sich für den Fall der Verwendung von Natriumhydroxid und Natriumhypochlorit durch die folgenden Formeln wiedergeben:

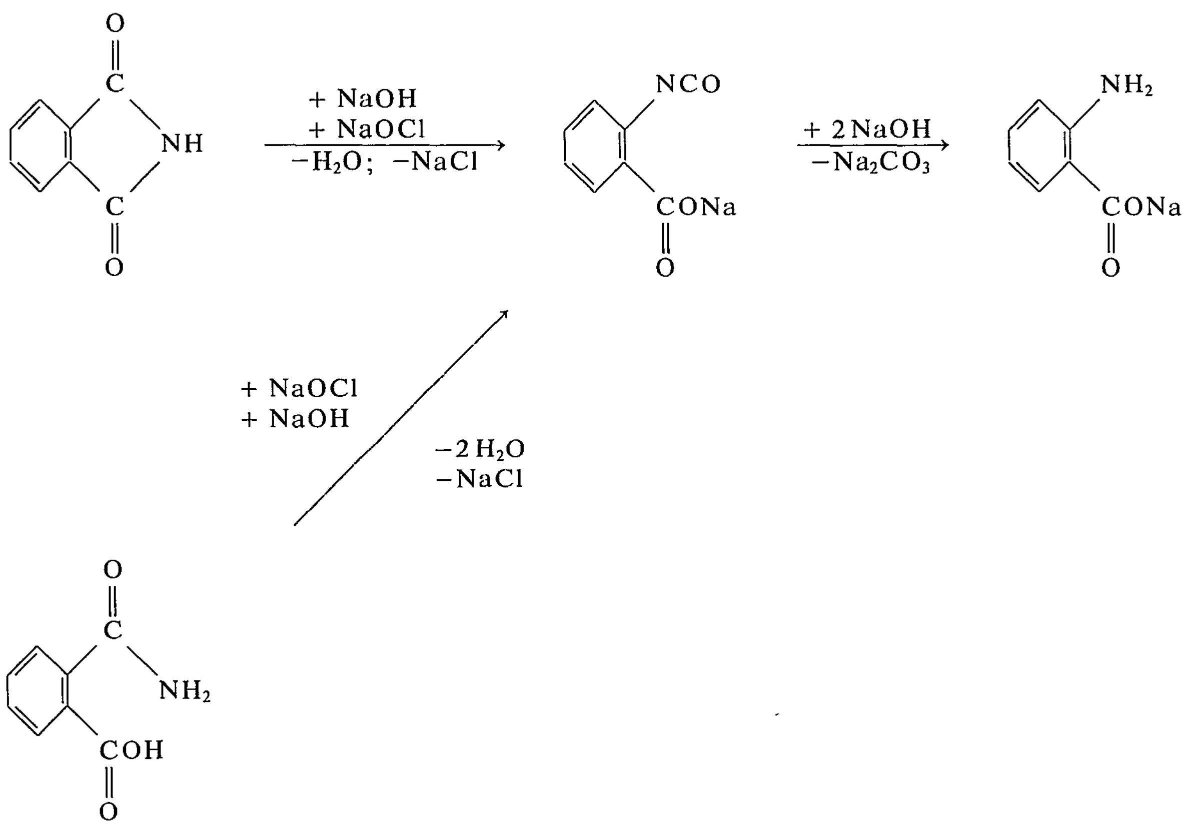


Im Vergleich zu den bekannten Verfahren liefert das Verfahren nach der Erfindung Anthranilsäure in besserer Reinheit auf einfacherem und wirtschaftlicherem Wege und besserer Raum-Zeit-Ausbeute an reinem Endstoff. Die in der Anthranilsäure als Verunreinigung enthaltenen farbigen Verbindungen werden in eine farblose, wasserlösliche Form überführt und abgetrennt. Die Reaktion kann ohne Ausbeuteverluste auch bei Temperaturen zwischen 80 und 100° C in der Reaktionsstufe der Umsetzung der zunächst gebildeten Phenylisocyanat-2-carbonsäure zur Anthranilsäure durchgeführt werden.

Diese vorteilhaften Ergebnisse werden auch ohne höchste Anforderungen an gleichmäßige Dosierung der Ausgangslösungen erzielt. Überschüssige Mengen an Hypochlorit beeinflussen die Ergebnisse nicht in deutlichem Maße. Schwankungen in der Konzentration der Nebenstoffe Ammoniak (Lösung des Alkalisalzes von Phthalimid- bzw. Phthalamidsäure) oder Natriumchlorat (Hypochloritlösung) spielen in bezug auf Ausbeute und Reinheit (Tönung) des Endstoffes keine wesentliche Rolle. Entsprechend hat die Bildung von Natriumchlorat oder anderen oxidierenden Nebenprodukten die in den ersten Stufen der Reaktion auftreten können, keinen wesentlichen Einfluß auf Menge und Farbe der erhaltenen Anthranilsäure. Das erfindungsgemäße Verfahren liefert im Hinblick auf die deutsche Auslegeschrift 2 000 698 gerade auch in industriellem Maßstab bei guter Ausbeute Anthranilsäure in gleichbleibend besserer Reinheit.

Die Umsetzung von phthalamidsaurem und/oder phthalimidsaurem Alkali mit Hypohalogeniten kann kontinuierlich im Reaktionsrohr in beliebiger Weise, zweckmäßig analog den in den vorgenannten Veröffentlichungen beschriebenen Verfahren, insbesondere den in der deutschen Auslegeschrift 1 950 281 oder deutschen Offenlegungsschrift 2 328 757 beschriebenen Verfahren,

durchgeführt werden. Eine besonders bevorzugte Ausführungsform der Umsetzung des erfindungsgemäßen Verfahrens besteht darin, daß man

a) Phthalimid und/oder Phthalamidsäure in wäßriger Alkalilauge mit einem Verhältnis von 3 bis 3,5 Mol Alkalihydroxid je Mol Phthalimid und/oder von 2 bis 2,5 Mol Alkalihydroxid je Mol Phthalamidsäure löst,
b) die so gebildete wäßrige Lösung von phthalamidsaurem und/oder phthalimidsaurem Alkali und eine wäßrige Lösung von Alkalihypochlorit in einer Mischvorrichtung mischt,
c) die erhaltene Mischung im ersten Teil eines Reaktionsrohres mit hoher Strömungsgeschwindigkeit bei 10 bis 54° C unter weitgehend adiabatischen Bedingungen umsetzt, anschließend
d) die aus dem ersten Teil des Reaktionsrohres mit hoher Strömungsgeschwindigkeit abströmende Umsetzungsmischung im zweiten Teil des genannten Rohres bei 55 bis 90° C zu Ende umsetzt und
e) aus der abströmenden alkalischen Umsetzungsmischung in üblicher Weise Anthranilsäure abtrennt.

Bevorzugt wird die Umsetzung in Gegenwart von Brom, Jod und/oder Halogenamiden der Formel

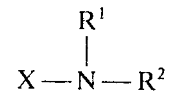 (I)

worin $R^1$ eine Sulfonsäuregruppe, einen Sulfonatrest, eine Sulfonamidgruppe bezeichnet, $R^2$ ein Wasserstoffatom, einen aliphatischen Rest, ein Chloratom oder Bromatom bedeutet, X ein Chloratom, Bromatom oder Wasserstoffatom bezeichnet, $R^1$ und $R^2$ darüber hinaus auch zusammen mit dem benachbarten Stickstoffatom Glieder eines heterocyclischen Restes, der mindestens eine dem Stickstoffatom benachbarte Sulfongruppe oder Phosponylgruppe der Formel

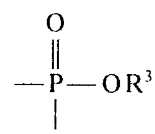

worin $R^3$ für ein Wasserstoffatom oder ein Alkaliatom steht, enthält, bezeichnen können, oder $R^1$ und $R^2$ zusammen auch den Rest

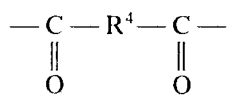

bedeuten können, worin $R^4$ für einen Alkylenrest, den Rest

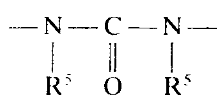

oder den Rest

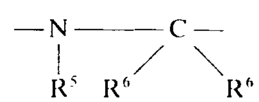

$R^5$ für ein Wasserstoffatom, ein Chloratom oder Bromatom und $R^6$ für einen aliphatischen Rest stehen, durchgeführt.

Als Ausgangsstoffe kann man phthalamidsaures und/oder phthalimidsaures Alkali und Hypohalogenite in wäßrigem Medium verwenden, in der Regel in Gestalt von entsprechenden wäßrigen, alkalischen Lösungen. Vorteilhaft gelangen wäßrige Lösungen von 10 bis 50 Gewichtsprozent Phthalimid und/oder Phthalamid zur Anwendung, die von 1 bis 1,1 Mol Alkalihydroxid je Mol Phthalimid/Phthalamid enthalten. Bevorzugt sind Natrium- und Kaliumhydroxid.

Die wäßrigen Hypohalogenitlösungen enthalten vorteilhaft von 5 bis 15, vorzugsweise von 12 bis 14 Gewichtsprozent Hypohalogenit und können zusätzlich von 0,2 bis 2,5 Mol, vorzugsweise 1 bis 2,1 Mol Alkalihydroxid je Mol Hypohalogenit enthalten. Im Ausgangsgemisch beider Ausgangsstoffe kommen im allgemeinen Mengen von insgesamt 0,9 bis 1,5, vorzugsweise 0,95 bis 1,1 Mol Hypohalogenit und zweckmäßig von insgesamt 0,2 bis 2,5 Mol, vorzugsweise 1 bis 2,1 Mol Alkalihydroxid (nicht eingerechnet das im Hypohalogenit enthaltene Alkali), bezogen auf 1 Mol Phthalimid und/oder Phthalamid, in Frage. Enthält die wäßrige Hypohalogenitlösung kein freies Alkalihydroxid, so werden

am Anfang oder im Laufe der Umsetzung zweckmäßig von 0,2 bis 2, vorzugsweise 1 bis 2 Mol Alkalihydroxid pro Mol Hypohalogenit zugeführt. Bevorzugtes Hypohalogenite sind Hypochlorite, insbesondere Alkalihypochlorite, z. B. das Natrium-, das Kaliumsalz, oder Erdalkalihypochlorite, z. B. das Calciumsalz.

In der vorgenannten, besonders bevorzugten Ausführungsform wird der Ausgangsstoff in Stufe a) in wäßriger Alkalilauge, zweckmäßig Kalilauge und insbesondere Natronlauge, gelöst. Vorteilhaft gelangen wäßrige Lösungen von 10 bis 50, bevorzugt 15 bis 30 Gewichtsprozent (bezogen auf die reine Wassermenge) Phthalimid und/oder Phthalamidsäure zur Anwendung, die von 3 bis 3,5, bevorzugt 3,1 bis 3,2 Mol Alkalihydroxid je Mol Phthalimid und/oder 2 bis 2,5, bevorzugt 2,1 bis 2,2 Mol Alkalihydroxid je Mol Phthalamidsäure enthalten. Die Lösung wird zweckmäßig bei einer Temperatur von −5 bis +50°C, vorzugsweise von 20 bis 30°C, drucklos oder unter Druck, kontinuierlich durchgeführt. Verwendet man Katalysatoren, z. B. die in der deutschen Offenlegungsschrift 2 357 749 oder vorteilhaft die in der deutschen Offenlegungsschrift 2 328 757 beschriebenen Katalysatoren, so werden sie zweckmäßig schon in Stufe a) dem Ausgangsstoff bzw. seiner Lösung zugesetzt. Man kann aber den Katalysator, zweckmäßig im Gemisch mit Wasser, auch dem Ausgangsgemisch getrennt oder zusammen mit dem Hypohalogenit zusetzen.

Als Katalysatoren kommen vorteilhaft Brom, Jod und/oder die vorgenannten Halogenamide I, im allgemeinen in einer Menge von 0,0001 bis 0,1, vorzugsweise von 0,001 bis 0,01 Mol Katalysator je Mol Phthalimid bzw. Phthalamidsäure in Betracht. An Stelle der genannten Stoffe können auch Verbindungen, die unter den Reaktionsbedingungen solche Stoffe bilden, verwendet werden, z. B. Bromide und Jodide an Stelle von Brom oder Jod. Zweckmäßig wählt man wasserlösliche Halogenide. Diese Halogenide kommen vorteilhaft in Gestalt ihrer Erdalkali- und insbesondere ihrer Alkalisalze in Frage, z. B. Calciumbromid, Calciumjodid, Magnesiumbromid, Magnesiumjodid, Lithiumbromid, Lithiumjodid und insbesondere Natrium- und Kaliumbromid oder -jodid. Bevorzugte Halogenamide I sind solche, in deren Formel $R^1$ eine Sulfonsäuregruppe, einen Sulfonatrest, insbesondere einen Alkalisulfonatrest wie Natriumsulfonat oder Kaliumsulfonat, eine Sulfonamidgruppe bezeichnet, $R^2$ ein Chloratom, ein Bromatom, einen Alkylrest mit 1 bis 4 Kohlenstoffatomen oder insbesondere ein Wasserstoffatom bedeutet, X ein Bromatom, ein Chloratom oder zweckmäßig ein Wasserstoffatom bezeichnet, $R^1$ und $R^2$ darüber hinaus auch zusammen mit dem benachbarten Stickstoffatom Glieder eines heterocyclischen, 5- oder 6gliedrigen Ringes, der mindestens eine dem Stickstoffatom benachbarte Sulfongruppe oder Phosphonylgruppe der Formel

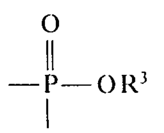

worin $R^3$ für ein Wasserstoffatom oder ein Alkaliatom, insbesondere ein Natriumatom oder Kaliumatom steht, enthält, bezeichnen können oder $R^1$ und $R^2$ zusammen den Rest

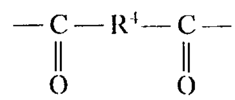

bedeuten können, worin $R^4$ für einen Alkylenrest mit 2 bis 4 Kohlenstoffatomen, den Rest

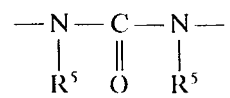

oder den Rest

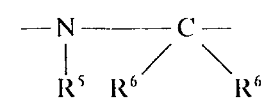

$R^5$ für ein Wasserstoffatom, ein Chloratom oder Bromatom und $R^6$ für einen Alkylrest mit 1 bis 4 Kohlenstoffatomen, insbesondere die Methylgruppe, stehen. An den vorgenannten heterocyclischen Ring kann noch ein Phenylkern anelliert sein. Vorteilhaft enthält der heterocyclische Rest 2 dem Stickstoffatom benachbarte Sulfon- oder Phosphongruppen oder zwei oder drei Sulfonamidogruppen oder Phosphonamidogruppen, insbesondere in demselben Ring, bei mehrkernigen heterocyclischen Resten. Vorgenannte bevorzugte Reste können noch unter den Reaktionsbedingungen inerte Gruppen oder Atome, z. B. Chloratome, Bromatome, Alkylgruppen mit 1 bis 4 Kohlenstoffatomen, den Phenylkern substituierende Carboxyl- oder Carboxylatgruppen, substituiert sein.

Als Katalysatoren kommen z. B. in Betracht: Glutarimid, Adipinsäureimid, Succinimid; vorzugsweise

Cyanursäure, 5,5-Dimethylhydantoin, Trisulfamid, N-Methyl-sulfaminsäure, Natriumtriimidometaphosphat; entsprechende Gemische vorgenannter Halogenamide I; besonders sind Sulfaminsäure und ihre Salze, zweckmäßig Alkalisalze wie das Natrium- oder Kaliumsalz, und insbesondere Sulfamid bevorzugt, gegebenenfalls im Gemisch mit vorgenannten Halogenamiden I.

Die wäßrigen Hypohalogenitlösungen der Stufe b) in der vorgenannten, besonders bevorzugten Ausführungsform enthalten vorteilhaft von 5 bis 15, insbesondere 12 bis 14 Gewichtsprozent Hypohalogenit und keine wesentlichen Mengen, höchstens bis zu 0,01 Mol überschüssiges Alkalihydroxid je Mol Phthalimid/Phthalamidsäure. Bevorzugte Alkalihypohalogenite sind Alkalihypochlorite, insbesondere das Kaliumsalz oder insbesondere Natriumhypochlorit. Im allgemeinen wird die Reaktion mit einem Verhältnis von Hypohalogenit von 1 bis 2, vorzugsweise 1 bis 1,2 Mol Hypohalogenit je Mol Phthalimid und/oder Phthalamidsäure durchgeführt. Vorteilhaft vermischt man den Ausgangsstoff in seiner wäßrigen, alkalischen Lösung vorgenannter Konzentration aus Stufe a) mit der Alkalihypochloritlösung in Stufe b) in vorgenanntem Mengenverhältnis in einer Mischungsvorrichtung. Solche Vorrichtungen können Mischzellen, Mischdüsen oder Kammern mit Rührwerken hoher Umdrehungszahl sein. Die Mischung wird in der Regel bei einer Temperatur zwischen 0 und 50°C, vorteilhaft zwischen 25 und 45°C, drucklos oder unter Druck, kontinuierlich durchgeführt.

Man kann die Ausgangsstoffe in dem Reaktionsrohr in einer allgemeinen Form bei einer Temperatur zwischen 0°C und 100°C, vorzugsweise zwischen 10 und 85°C, drucklos oder unter Druck, kontinuierlich oder diskontinuierlich umsetzen. Die Reaktion kann wie folgt durchgeführt werden: Ein Gemisch von Ausgangsstoffen, Katalysator, Alkali und Wasser wird während 1 bis 4000 Sekunden bei der Reaktionstemperatur gehalten. In einer weiteren Ausführungsform, die gleichzeitig den besonders einfachen und vorteilhaften Betrieb von Anlagen nach dem erfindungsgemäßen Verfahren illustriert, wird das Ausgangscarbonsäureamid, z. B. Phthalamidsäure, zuerst aus Carbonsäureanhydrid, Ammoniak und gegebenenfalls Alkalihydroxid bei einer Temperatur von in der Regel 20 bis 80°C hergestellt und das so gebildete Reaktionsgemisch ohne Isolierung des Reaktionsproduktes direkt als Ausgangsstoff nach dem erfindungsgemäßen Verfahren umgesetzt. Zweckmäßig kann man die Umsetzung auch in den vorgenannten Stufen a) bis d) des Verfahrens nach der deutschen Auslegeschrift 1 950 281 durchführen.

In der vorgenannten, besonders bevorzugten Ausführungsform wird die Reaktion vorteilhaft in 2 Reaktionsräumen (Stufe c) und d)) unter weitgehender Vermeidung der Rückmischung in beiden Räumen und weitgehend adiabatisch in der ersten Stufe durchgeführt. Die Umsetzung erfolgt in 2 Reaktionsschritten, der Reaktionsstufe c), der Umsetzung des Ausgangsstoffes über das N-chlor-phthalamidsaure Alkalisalz zum phenylisocyanat-2-carbonsauren Alkalisalz und der folgenden Stufe d) der Umsetzung des Alkalisalzes zur Anthranilsäure. Die erste Reaktionsstufe wird weitgehend unter adiabatischen Bedingungen durchgeführt, die entstehende Reaktionswärme erwärmt dabei das Umsetzungsgemisch in der Regel auf eine Temperatur zwischen 20 und 50°C. Aus der Mischungsvorrichtung gelangt das Reaktionsgemisch in den Reaktionsraum der ersten Reaktionsstufe (Stufe c)), der aus einem vorteilhaft engen Reaktionsrohr besteht, und von dort nach der Umsetzung in den Reaktionsraum der folgenden Stufe (Stufe d)). Mischungsvorrichtung, der Reaktionsraum der ersten Stufe und die Lösungen der Ausgangsstoffe brauchen nicht gekühlt zu werden. Ein vorteilhaftes Merkmal des Verfahrens in der vorgenannten, besonders bevorzugten Ausführungsform ist die weitgehende Vermeidung der Rückmischung in Stufe c), ein rascher Entzug des Reaktionsgemisches aus c) und seine Zuführung — unter weitgehender Vermeidung der Rückmischung — in die Stufe d). Zweckmäßig stellt man durch einen engen Querschnitt des Reaktionsrohres der ersten Stufen und Verwendung entsprechender Transportpumpen eine hohe Strömungsgeschwindigkeit des Reaktionsgemisches ein. Als Pumpen können z. B. Strahl-, Rotations-, Kreiskolben-, Wälzkolben-, Schraubenkolben-, Exzenter-, Flügel-, Kreisel-, Axial-, Propellerpumpen verwendet werden. In einer bevorzugten Ausführungsform des Verfahrens werden die Strömungsgeschwindigkeiten durch Querschnitt und Länge des Reaktionsrohres bestimmt. Vorteilhaft sind z. B. Reaktorquerschnitte von 10 bis 10 000 $mm^2$ und Strömungsgeschwindigkeiten von 0,1 bis 10, insbesondere 0,2 bis 3 m/sec, vorzugsweise 0,5 bis 1,2 m/sec. Bei diesen Geschwindigkeiten wird in der Regel in einer Verweilzeit von 0,4 bis 40, vorzugsweise von 0,6 bis 20 Sekunden der Ausgangsstoff in der Stufe c) weitgehend über das am Stickstoffatom chlorierte Phthalsäuremonoamid zum Alkalisalz der Phenylisocyanat-2-carbonsäure umgesetzt. Das gebildete Alkalisalz wird, bedingt durch die hohe Strömungsgeschwindigkeit, dem Reaktionsraum der Stufe c) sofort entzogen, der folgenden Stufe zugeführt und dort, im allgemeinen mit einer Verweilzeit von 0,3 bis 150, vorzugsweise von 0,3 bis 40 Sekunden, zur Anthranilsäure umgesetzt. Die hohe Strömungsgeschwindigkeit verhindert gleichzeitig weitgehend eine Rückmischung innerhalb der gesamten Umsetzung des Reaktionsgemisches. Insbesondere wird die Rückmischung des gebildeten Endstoffes mit dem Reaktionsgemisch der Stufe c) vermieden und damit die Bildung von Nebenprodukten durch Umsetzung des Hypohalogenits bzw. des N-chlorierten Phthalsäuremonoamids mit dem Endstoff und/oder durch entsprechende Umsetzungen in den Gemischen der Stufen c) und d) unterdrückt. Die Reaktion wird in Stufe c) bei einer Temperatur von 10 bis 54°C, vorzugsweise zwischen 20 und 54°C, insbesondere zwischen 30 und 54°C, in der Stufe c) von 55 bis 90°C, vorzugsweise von 60 bis 90°C, drucklos oder

unter Druck durchgeführt. Die Beendigung der Reaktionsstufe c) wird in der Regel durch einen Temperaturanstieg von 20 bis 30° C auf etwa 42 bis 53° C angezeigt. Querschnitt, Strömungsgeschwindigkeit und Temperatur der Ausgangslösungen bestimmten im allgemeinen die Länge der Rohrstrecke, in der die erste Reaktionsstufe c) durchgeführt wird. Beispielsweise ist die Stufe c) bei einem Rohrquerschnitt von 2200 $mm^2$ und bei einer Strömungsgeschwindigkeit von etwa 1 m/sec und bei einer Ausgangstemperatur von etwa 40° C häufig nach etwa 1,5 Meter Länge des Reaktionsrohres beendigt. Am Ende der Reaktionsfolge wird das Reaktionsgemisch entnommen. Die Isolierung der Endstoffe aus den alkalischen Lösungen kann durch Ausfällen mit Säuren, z. B. Salzsäure oder Schwefelsäure, und nachfolgender Filtration vorgenommen werden.

Das Verfahren nach der Erfindung geht von der Beobachtung aus, daß nicht die Reduktionsmittel generell die Reinheit des Endstoffes verbessern, sondern für die Erzielung optimaler Mengen sehr reinen Endstoffes die Behandlung durch zwei unterschiedliche Reduktionsmittel notwendig ist; es ist ein entscheidendes erfindungsgemäßes Merkmal, daß im ersten Schritt noch eine größere Zahl von Reduktionsmitteln, aber keinesfalls Alkalidithionit und/oder Alkaliformaldehydsulfoxylat, in Frage kommt, im zweiten Schritt aber allein Alkalidithionit und/oder Alkaliformaldehydsulfoxylat verwendet werden müssen. Diese spezifische Folge selektiv wirkender Reduktionsmittel ist in ihren vorteilhaften Ergebnissen für das erfindungsgemäße Verfahren überraschend.

Im ersten Behandlungsschritt kann eine große Zahl von in Wasser und/oder Alkalien löslichen oder mit ihnen mischbaren Reduktionsmitteln verwendet werden. Geeignet sind beispielsweise Hydride wie Natriumborhydrid, Lithiumtriäthoxy-aluminiumhydrid; reduzierende Schwefelverbindungen wie Natriumsulfid, Natriumhydrogensulfid, Ammoniumsulfid, schweflige Säure, Schwefeldioxid, Natriumthiosulfat, Thioharnstoffdioxid; Hydrazin und seine Salze, z. B. das Sulfat oder Chlorid; Glucose. Bevorzugte Reduktionsmittel sind Natriumsulfit und Natriumbisulfit. Das Reduktionsmittel kann in stöchiometrischem Verhältnis oder im Überschuß zum eingesetzten Hypohalogenit, vorzugsweise in einer Menge von 0,0005 bis 0,2 insbesondere von 0,005 bis 0,1 Äquivalenten Reduktionsmittel je Mol eingesetztes Hypohalogenit, verwendet werden. Zweckmäßig kommen Lösungen des Reduktionsmittels in Wasser, z. B. 10- bis 40gewichtsprozentige, wäßrige Natriumbisulfitlösungen, in Betracht.

Alkalidithionit und/oder Alkaliformaldehydsulfoxylat kann in stöchiometrischem Verhältnis oder im Überschuß zum eingesetzten Hypohalogenit, vorzugsweise von 0,0005 bis 0,2, insbesondere von 0,005 bis 0,1 Äquivalenten Alkalidithionit und/oder Alkaliformaldehydsulfoxylat je Mol eingesetztes Hypochlorit, verwendet werden. Zweckmäßig kommen Lösungen des Reduktionsmittels in Wasser, z. B. 1- bis 20gewichtsprozentige, wäßrige Natriumdithionitlösungen, in Betracht. Ein Verhältnis von 0,1 bis 1, insbesondere von 0,2 bis 0,8 Äquivalent Alkalidithionit und/oder Alkaliformaldehydsulfoxylat je Mol Reduktionsmittel des ersten Behandlungsschrittes ist vorteilhaft. Von den Alkalidithioniten verwendet man bevorzugt Natriumdithionit, von den Alkaliformaldehydsulfoxylaten bevorzugt Natriumformaldehydsulfoxylat. Natriumdithionit kann auch in Gestalt von Hydrosulfit, Natriumformaldehydsulfoxylat (Hydroxymethansulfinsaures Natrium) auch in Gestalt des im Handel unter der Bezeichnung ®Rongalit erhältlichen Produktes verwendet werden.

Die Behandlung in beiden Schritten wird in der Regel bei einer Temperatur zwischen 0 und +100° C, vorzugsweise bei einer Temperatur von 10 bis 90° C, insbesondere im ersten Behandlungsschritt von 55 bis 90° C, insbesondere im zweiten Behandlungsschritt von 88 bis 5° C, diskontinuierlich oder zweckmäßig kontinuierlich, drucklos oder unter Druck, durchgeführt. Man kann beide Schritte während der Reaktion, z. B. den ersten Schritt nach Beginn oder in der ersten Hälfte der Reaktionszeit, den zweiten Schritt in der zweiten Hälfte der Reaktionszeit oder am Ende der zweiten Hälfte der Reaktionszeit durchführen; in vorgenannter Weise wählt man dann für den ersten Schritt die Stufe b) der deutschen Auslegeschrift 1 950 281 oder in der vorgenannten, bevorzugten Ausführungsform die Stufe c), für den zweiten Schritt die Stufe c) des Verfahrens nach der deutschen Auslegeschrift 1 950 281 oder in der vorgenannten, bevorzugten Ausführungsform die Stufe d). Zweckmäßiger wird man jedoch während der Reaktion beide Behandlungsschritte in die zweite Hälfte der Reaktionszeit legen, z. B. den ersten Behandlungsschritt zu Beginn oder während der zweiten Hälfte der Reaktionszeit oder zu Beginn oder während der Stufe c) nach dem Verfahren der deutschen Auslegeschrift 1 950 281 oder zu Beginn oder während der Stufe d) in der vorgenannten, bevorzugten Ausführungsform legen und den zweiten Behandlungsschritt kurz vor dem Ende oder am Ende der zweiten Hälfte der Reaktionszeit, d. h. kurz vor dem Ende oder am Ende der Stufe c) nach dem Verfahren der deutschen Auslegeschrift 1 950 281 oder kurz vor dem Ende oder am Ende der Stufe d) in der vorgenannten, bevorzugten Ausführungsform legen. Ebenfalls kann man zweckmäßig beide Behandlungsschritte nach der Reaktion durchführen, z. B. den ersten Behandlungsschritt direkt nach Beendigung der Reaktion oder der Stufe c) nach dem Verfahren der deutschen Auslegeschrift 1 950 281 oder der Stufe d) in der vorgenannten, bevorzugten Ausführungsform, darauf den zweiten Behandlungsschritt durchführen und schließlich die Abtrennung des Endstoffes mit dem Ansäuern des Behandlungsgemisches beginnen. Besonders bevorzugt ist die folgende Ausführungsform: Der erste Behandlungsschritt beginnt kurz vor Ende oder am Ende der Reaktion, d. h. der Stufe c) nach dem Verfahren der deutschen Offenlegungsschrift 1 950 281 oder der Stufe d) in der vorgenannten, bevorzugten Ausführungsform, zweckmäßig zwischen 0,2 und 0 Sekunden vor dem Endpunkt der Reaktion, und wird nach Zugabe des Reduktionsmittels zweckmäßig so lange fortgesetzt, daß

insgesamt ab Beginn der Zugabe des Reduktionsmittels eine Verweilzeit von 0,2 bis 40, insbesondere von 1 bis 15 Sekunden des Reaktionsgemisches zusammen mit dem Reduktionsmittel eingehalten wird. Anschließend beginnt man vorteilhaft mit dem zweiten Behandlungsschritt und der Zugabe von Alkalidithionit und/oder Alkaliformaldehydsulfoxylat und hält zweckmäßig eine Verweilzeit von 0,2 bis 120, insbesondere von 0,5 bis 60 Sekunden ab Beginn der Zugabe von Alkalidithionit und/oder Alkaliformaldehydsulfoxylat ein. Man kann in beiden Schritten auch langsam das Reduktionsmittel bzw. Alkalidithionit und/oder Alkaliformaldehydsulfoxylat während der genannten, vorgenannten Verweilzeit zugeben; jedoch ist raschere Zugabe und anschließende Verweilzeit ohne Zugabe zweckmäßiger. Der erste Behandlungsschritt erfolgt zweckmäßig noch etwa bei der Temperatur des Reaktionsgemisches vor oder bei Ende der Reaktion, oder der vorgenannten Temperaturen von Stufe c) nach dem Verfahren der deutschen Auslegeschrift 1 950 281 und Stufe d) in der vorgenannten, bevorzugten Ausführungsform. Das Reaktionsgemisch wird dann vorteilhaft im zweiten Behandlungsschritt noch kurzfristig, z. B. 0,2 bis 40 Sekunden bei dieser Temperatur gehalten und dann abgekühlt, zweckmäßig auf 5 bis 25° C. Die Geschwindigkeit des Zusatzes in beiden Behandlungsschritten richtet sich in der Regel nach der Strömungsgeschwindigkeit des Reaktionsgemisches, wobei die Konzentration der zugesetzten Lösung und das vorgenannte Verhältnis Reduktionsmittel zu Ausgangshypohalogenit zu berücksichtigen ist. Die Dosierung der Reduktionslösung kann in beliebiger Weise, über Kammern mit Rührwerken, Mischdüsen oder vorzugsweise über eine Mischzelle, erfolgen. Die Behandlung kann nach Zugabe des Reduktionsmittels im Reaktionsrohr mit hoher Strömungsgeschwindigkeit, z. B. mit 0,2 bis 3 m/sec, oder auch ohne Verminderung der Ausbeute in einem Reaktionsrohr beliebiger Dimensionierung erfolgen und zweckmäßig auch die weitere Behandlung in den zwei Schritten in der Verlängerung des Reaktionsrohres bzw. entsprechend kontinuierlich in nachfolgenden Behandlungsrohrreaktoren durchgeführt werden. Vorteilhaft erfolgt die Behandlung im ersten und zweiten Schritt bei einem pH von 9 bis 14.

Nach den Behandlungsschritten folgt zweckmäßig die Abtrennung des Endstoffes in üblicher Weise, z. B. durch Einstellen des pH-Wertes auf 4,2 und Abfiltrieren der Anthranilsäure oder durch Extraktion mit einem organischen Lösungsmittel wie Benzol oder chlorierten Kohlenwasserstoffen wie Trichloräthylen.

Die nach dem Verfahren der Erfindung herstellbaren Verbindungen sind wertvolle Ausgangsstoffe für die Herstellung von Farbstoffen, Pflanzenschutzmitteln und Riechstoffen. Bezüglich der Verwendung wird auf die genannten Patentschriften und Ullmanns Encyklopädie der technischen Chemie (4. Auflage), Band 8, Seite 375, verwiesen.

Die in den folgenden Beispielen angeführten Teile bedeuten Gewichtsteile. Sie verhalten sich zu den Volumenteilen wie Kilogramm zu Liter.

### Beispiel 1

Man verwendet eine Anlage, die aus einer Mischdüse und einem Reaktorrohr mit einem Reaktionsrohrteil (Reaktionsraum) von 1,1 Meter Länge und 53 Millimeter Innendurchmesser besteht. 590 Teile flüssiges Phthalimid werden stündlich in einer Mischdüse kontinuierlich in 2103 Teilen wäßriger, 25gewichtsprozentiger Natronlauge und 4752 Teilen Wasser gelöst, und stündlich 18 Teile 30gewichtsprozentige, wäßrige Lösung des Natriumsalzes der Sulfaminsäure zugeführt. Die gebildete Lösung wird stündlich in der Mischdüse mit 2100 Teilen wäßriger Natriumhypochloritlösung (290 Teilen Natriumhypochlorit; 13,8 Gewichtsprozent akt. Chlor) bei 42°C gemischt. Die Strömungsgeschwindigkeit im nachfolgenden Reaktionsrohrteil beträgt 1,1 Meter pro Sekunde. Die Verweilzeit beträgt eine Sekunde. Das Reaktionsgemisch wird im ersten Teil des Reaktionsraumes (Stufe c)) (0,3 m) weitgehend adiabatisch (von 42 bis 53°C) umgesetzt, wobei im verbleibenden Reaktionsraum (Stufe d)) eine Selbsterwärmung bis 89° C auftritt. Das Gemisch wird kontinuierlich stündlich in einem Abstand von 0,90 Meter ab Eingang des Reaktionsrohres (Anfang der Stufe c)) mit 25 Teilen 40gewichtsprozentiger, wäßriger Natriumbisulfitlösung, versetzt. 10 Sekunden nach Zugabe verweilt das Gemisch bei derselben Strömungsgeschwindigkeit im Reaktionsrohr bei 88°C. Dann werden 15 Teile 20prozentiger, wäßriger Natriumdithionitlösung stündlich zugegeben. Die Lösung wird auf 10°C abgekühlt und mit Salzsäure auf pH-Wert 4,2 eingestellt, abgesaugt und der Filterrückstand mit Wasser gewaschen und getrocknet. Die Verweilzeit des zweiten Behandlungsschrittes (von Beginn der Zugabe des Dithionits bis zur Zugabe der Säure) beträgt 40 Sekunden, die Verweilzeit ab Beginn der Abkühlung 30 Sekunden. Man erhält stündlich 535 Teile (97% der Theorie) Anthranilsäure (99,9%) vom Fp 146,1° C; Raum-Zeit-Ausbeute: 268 Teile pro Stunde und Liter.

### Beispiel 2

Man führt die Umsetzung analog Beispiel 1, aber unter Zugabe von 40 Teilen pro Stunde Natriumformaldehydsulfoxylat an Stelle von Natriumdithionit durch. Anthranilsäure wird in gleicher Ausbeute und Reinheit erhalten.

Beispiel 3
(Vergleich)

a) Führt man die Umsetzung analog Beispiel 1, aber unter Zusatz von stündlich 25 Teilen 40gewichtsprozentiger, wäßriger Natriumbisulfitlösung im zweiten Behandlungsschritt an Stelle von Natriumdithionit durch, so erhält man stündlich 530 Teile (96% der Theorie) Anthranilsäure von einem Gehalt von 98,9 Prozent und Fp 145,7° C. Raum-Zeit-Ausbeute: 265 Tage pro Stunde und Liter.

b) Führt man die Umsetzung analog Beispiel 1, aber unter Zugabe von stündlich 15 Teilen 20gewichtsprozentiger, wäßriger Natriumdithionitlösung im ersten Behandlungsschritt an Stelle von Natriumbisulfit und unter Zusatz von stündlich 25 Teilen 40gewichtsprozentiger, wäßriger Natriumbisulfitlösung im zweiten Behandlungsschritt an Stelle von Natriumdithionit durch, so erhält man stündlich 525 Teile (95% der Theorie) Anthranilsäure von einem Gehalt von 99 Prozent und Fp 145,9° C. Raum-Zeit-Ausbeute: 263 Teile pro Stunde und Liter.

c) Führt man die Umsetzung analog Beispiel 1, aber unter Zugabe von stündlich 15 Teilen 20gewichtsprozentiger, wäßriger Natriumdithionitlösung im ersten Behandlungsschritt und unter Zusatz von stündlich 15 Teilen 20gewichtsprozentiger, wäßriger Natriumdithionitlösung im zweiten Behandlungsschritt durch, so erhält man stündlich 530 Teile (96% der Theorie) Anthranilsäure von einem Gehalt von 98,9 Prozent und Fp 145,9° C. Raum-Zeit-Ausbeute: 265 Teile pro Stunde und Liter.

d) Die nach Beispiel 3a)—c) erhaltene Anthranilsäure weist eine braunstichige Tönung auf und enthält, wie eine Lösung in verdünnter Salzsäure zeigt, unlösliche Rückstände. Extinktionsmessungen mit einer 5gewichtsprozentigen Anthranilsäurelösung in 1 n-Salzsäure bei einem Licht der Wellenlänge von 430 Nanometer ergeben folgende Werte:

| Beispiel | Extinktion |
|---|---|
| 1 | 0,1 |
| 2 | 0,11 |
| 3a) | 0,2 |
| 3b) | 0,2 |
| 3c) | 0,2 |

**Patentanspruch**

Verfahren zur kontinuierlichen Herstellung von Anthranilsäure durch Umsetzung von phthalamidsaurem und/oder phthalimidsaurem Alkali mit Hypohalogeniten in wäßrigem Medium in einem Reaktionsrohr und unter Behandlung des die gebildete Anthranilsäure enthaltenden Reaktionsgemisches mit Reduktionsmitteln, dadurch gekennzeichnet, daß man während der Reaktion oder nach Beendigung der Reaktion die Behandlung mit Reduktionsmitteln in 2 Schritten durchführt, wobei man in einem ersten Schritt das Reaktionsgemisch, das die erhaltene Anthranilsäure enthält, mit von Alkalidithionit und/oder Alkaliformaldehydsulfoxylat unterschiedlichen Reduktionsmitteln behandelt und in einem zweiten Schritt die behandelte Anthranilsäure mit Alkalidithionit und/oder Alkaliformaldehydsulfoxylat behandelt.

**Claim**

A process for the continuous manufacture of anthranilic acid by reacting an alkali metal phthalamate and/or phthalimidate with a hypohalite in an aqueous medium in a reaction tube, and with the treatment oft the reaction mixture, containing the anthranilic acid formed, with reducing agents, characterized in that, during the reaction or after completion of the reaction, the treatment with reducing agents is carried in two steps, in the first step, the reaction mixture containing the anthranilic acid obtained being treated with reducing agents different from an alkali metal dithionite and/or an alkali metal formaldehyde-sulfoxylate, and in the second step, the treated anthranilic acid being treated with an alkali metal dithionite and/or an alkali metal formaldehyde-sulfoxylate.

**Revendication**

Procédé pour la préparation en continu de l'acide anthranilique par réaction d'un sel de métal alcalin du mono-amide phtalique et(ou) de l'imide phtalique en milieu aqueux, dans un réacteur tubulaire, avec un hypohalogénite, suivie du traitement du mélange réactionnel contenant l'acide anthranilique formé par des agents réducteurs, caractérisé en ce que le traitement avec les agents réducteurs est effectué en deux phases, soit pendant la réaction, soit après la fin de celle-ci, la première phase consistant à traiter le mélange réactionnel contenant l'acide anthranilique avec des agents réducteurs autres que les dithionites de métaux alcalins et(ou) les formaldéhyde-sulfoxylates de métaux alcalins et la deuxième phase comportant le traitement de l'acide anthranilique traité avec un dithionite et(ou) un formaldéhyde-sulfoxylate de métal alcalin